# Europäisches Patentamt
# European Patent Office
# Office européen des brevets

(19)

(11) Veröffentlichungsnummer: **0 172 443**
**B1**

(12)

# EUROPÄISCHE PATENTSCHRIFT

(45) Veröffentlichungstag der Patentschrift:
03.02.88

(21) Anmeldenummer: 85109356.7

(22) Anmeldetag: 25.07.85

(51) Int. Cl.⁴: **C 12 M 1/00**, C 02 F 3/28

(54) Verfahren und Vorrichtung zur anaeroben Behandlung von organischen Substraten zur Erzeugung von Biogas.

(30) Priorität: 28.07.84 DE 3427976

(43) Veröffentlichungstag der Anmeldung:
26.02.86 Patentblatt 86/9

(45) Bekanntmachung des Hinweises auf die Patenterteilung:
03.02.88 Patentblatt 88/5

(84) Benannte Vertragsstaaten:
AT BE CH DE FR GB IT LI LU NL SE

(56) Entgegenhaltungen:
AT-A-369 782
DE-A-3 102 739
DE-A-3 232 530
DE-C-3 134 980

(73) Patentinhaber: Harrendorf, Heinz, Rhönweg 7,
D-3000 Hannover 1 (DE)

(72) Erfinder: Harrendorf, Heinz, Rhönweg 7, D-3000
Hannover 1 (DE)

(74) Vertreter: König, Norbert, Dipl.- Phys. Dr.,
Patentanwälte Leine & König Burckhardtstrasse
1, D-3000 Hannover 1 (DE)

EP 0 172 443 B1

**Beschreibung**

Die Erfindung betrifft ein Verfahren zur anaeroben Behandlung von organischen Substraten zur Erzeugung von Biogas gemäß Oberbegriff des Anspruchs 1 sowie eine Vorrichtung zur Durchführung dieses Verfahrens gemäß Oberbegriff des Anspruchs 3.

Seit der Energiekrise 1973 wurde ein Umdenkprozeß in der Industrie und Landwirtschaft eingeleitet, da die Energieversorgung für viele Betriebe zu einer Rentabilitätsfrage wurde.

Durch neue Technologien wie Wärmerückgewinnung, Energieverbund, Mikroelektronik, Einsatz von wärmedämmenden Baustoffen usw. konnten bereits substantielle Energien eingespart werden. Als alternative Energiequellen wie Erd- und Sonnenwärme stehen insbesondere für die hier beschriebene Erfindung Abfallprodukte in landwirtschaftlichen und industriellen Betrieben zur Diskussion. Diese Abfallprodukte fallen als sogenannte Biosubstrate in Form von flüssigen Exkrementen von Tieren -auch Gülle genannt- in landwirtschaftlichen Betrieben, landwirtschaftlichen Brennereien und Schlachthöfen an, wobei die pflanzlichen Substrate und Biomassen als Schlempe bzw. Abwasser mit organisch hoher Schmutzfracht sowie als Gemüseabfälle in gewerblichen Brennereien, Konserven- und Lebensmittelfabriken anfallen.

In den Betrieben, wo der Flüssigmist von Tieren bzw. Abwasser mit einem hohen Anteil an organischer Schmutzfracht zur Verfügung steht, ist die Möglichkeit gegeben, eine Biogasanlage zu erstellen, um einen entsprechenden Anteil zur Verbrauchsdeckung von Heizöl, Erdgas und elektrischer Energie zu leisten.

Es handelt sich hier um eine Bio-Energieanlage, also eine Anlage, die Methangas aus den vorgenannten Substraten erzeugt, das sowohl in einem Wasser- oder Dampfkessel als auch in einem Ottomotor, der einen Drehstromgenerator antreibt, verbrannt werden kann.

Es ist bekannt, daß Bakterien abbaufähige organische Substanzen, also Kohlehydrate, Proteine und Fette sowohl unter Sauerstoffabschluß als auch bei Vorhandensein von Sauerstoff zerlegen bzw. abbauen können. Vorliegend handelt es sich um einen Prozeß, der bei völliger Dunkelheit und Sauerstoffabschluß in einem Substrat stattfindet; man spricht hier auch von einer anaeroben Fermentation.

Es steht der mesophile Temperaturbereich 32 - 38°C zur Diskussion, wobei der thermophile Temperaturbereich 53 - 58°C vorwiegend bei Substraten mit gefährlichen pathogenen Keimen infrage kommt. An der Biomethanisierung der organischen Substanzen, die in vier Phasen abläuft, sind nach den derzeitigen Erkenntnissen drei verschiedene Bakteriengruppen beteiligt. In der hydrolytischen und säurebildenden Prozeßphase werden von den fakultativen anaeroben Bakterien mit fermentativer bzw. acidogener Charakteristik die organisch hochmolekularen Verbindungen mit Hilfe von Enzymen zu niedermolekularen Verbindungen wie Aminosäuren, Glycerin und Fettsäuren hydrolysiert, wobei in einer weiteren Phase aus den Hydrolyseprodukten Essig-, Butter-, Propion-, Valerian-, Capron-, Ameisen- und Milchsäure sowie Ethanol, Wasserstoff, Kohlendioxyd, Ammoniak und Schwefelwasserstoff produziert werden.

Die Zusammensetzung dieser Stoffwechselprodukte wird allerdings in entsprechendem Maße durch den Wasserstoffpartialdruck beeinflußt. Während bei einem niedrigen $H_2$-Druck bzw. einer mittleren Raumbelastung mehr Propionsäure gebildet wird, entsteht bei einem hohen $H_2$-Druck bei mittlerer Raumbelastung mehr Valeriansäure. Darüber hinaus wirkt ein hoher Wasserstoffpartialdruck als Inhibitor bei der Katalisierung von Essigsäure. Die essigsäure- und wasserstoffbildende Prozeßphase wird von den obligaten anaeroben Bakterienstämmen, jedoch mit acetogener Charakteristik, übernommen, wobei von dieser Gruppe die langkettigen Fettsäuren, organischen Säuren, Alkohole und Aromaten zu Essigsäure, Wasserstoff und Kohlendioxyd abgebaut werden.

Das Ausgangsprodukt für die Methanbakterien -es handelt sich auch hier um die obligaten Anaerobier- welche eine methanogene Charakteristik aufweisen, sind in erster Linie Essigsäure, Wasserstoff und Kohlendioxyd, die in letzter Instanz zu Methangas ($CH_4$) und Kohlendioxyd ($CO_2$) umgewandelt werden. Es ist hierbei interessant, daß die Methanbakterien zum Wachstum ein Redoxpotential von -330 m Volt benötigen, sowie in einer räumlich engen Symbiose mit den acetogenen Bakterien leben, ph-Wert-empfindlich sind und keine schwankenden Substrattemperaturen vertragen. Darüber hinaus ist diese Lebensgemeinschaft besonders empfindlich gegen einwirkende Scherkräfte, z. B. durch Rührwerke. Auch die Anwesenheit von gelöstem Sauerstoff ist für die Methanbakterien teilweise tödlich.

Da der $H_2$ - Partialdruck im Substrat niedrig sein soll, müssen Kopplungsreaktionen stattfinden, wie z. B.

$$4 H^+ + 4 NAD(P)H \rightarrow 4 NAD(P)+ + 4 H_2 + 18,4 E$$
$$4 H_2 + CO_2 \rightarrow CH_4 + 2 H_2O - 33,2 E$$

**Gesamtreaktion:**

$$4 H^+ + 4 NAD(P)H + CO_2 \rightarrow 4 NAD(P)+ + CH_4 + 2 H_2O - 14,8 E$$

NAD = Nicotinamidadenindinucleotid
E = Freie Energie (KJ/Mol).

Ein besonderer Aspekt für die Reduzierung des Wasserstoffpartialdruckes sind die in den Methanbakterien gefundenen Coenzyme, insbesondere NAD, welche mittels eines chemieosmotischen Stoffwechselprozesses für den Abbau des $H_2$-Druckes sorgen.

Durch eine Reduzierung des H₂-Druckes erfolgt auch ein entsprechender Einfluß auf die freie Energie, d.h., daß mit abnehmendem H₂-Partialdruck sich die Umsetzung von organischen Verbindungen von einem endergonen zu einem exergonen Prozeßablauf ändert.

Da es sich bei den ersten Prozeßphasen um einen sogenannten sauren biologischen Prozeßablauf handelt und die Methanbakterien ihre Lebenstätigkeit nur in einem schwach alkalischen Medium entfalten können, ist es daher von Bedeutung, daß die einzelnen Prozeßphasen in einem sogenannten Gleichgewicht stehen. Sollten sich nämlich die organischen Säuren anhäufen, was auch an dem gemessenen ph-Wert festgestellt werden kann, so ist dies ein Zeichen dafür, daß aus irgendeinem Grund die Abbautätigkeit der säureproduzierenden Bakterien die der säureverbrauchenden Bakterien überwiegt und damit das Gleichgewicht gestört ist, d.h., daß die Methanbakterien gelähmt werden und damit der Faulprozeß umkippt, soweit nicht entsprechende Gegenmaßnahmen zur Unterstützung der letzten Prozeßphase getroffen werden.

Sind die organischen Stoffe im Substrat mit einem entsprechenden Abbaugrad biomethanisiert, ist die technische Faulgrenze erreicht, d.h., daß das Substrat abgeschlammt bzw. ausgetragen wird. Das abgeschlammte Substrat -auch Faulschlamm genannt- ist fast geruchlos, weitgehend hygienisiert, hat einen entsprechenden Nährstoffreichtum und kann als Dünger weiter verwendet werden.

Nach den bisherigen Gesichtspunkten hat sich für den Bau von Biogasanlagen in erster Linie der sogenannte Durchlaufreaktor -auch Anflow-Reaktor genannt- herauskristallisiert. Dieser Reaktor arbeitet einstufig, wobei das Substrat durchgemischt wird. In diesem Reaktor laufen alle vier geschilderten bio-technologischen Phasen in einem Raum uniter gleichen physikalischen Bedingungen ab. Es ist verständlich, daß bei diesem Verfahren keine optimalen Lebensbedingungen für die einzelnen Bakterienstämme vorhanden sind. Darüber hinaus besteht die Gefahr, daß der Reaktor sauer wird d.h., der ph-Wert sinkt auf einen für die Methanbakterien nicht mehr zulässigen Wert unter 7 herab, da eine Überproduktion an Säuren vorliegt.

Auch durch den Eintrag an toxischen Stoffen wie Sulfonamide, Kupfer-, Zink- und Chromverbindungen usw. sowie des Sauerstoffinhibitors wird die Stoffwechselproduktion der entsprechenden Abbauphase gestört. Eine weitere Störung der Stoffwechselproduktion kann bei einem biologischen Abbau von Substraten, z. B. Schlempe mit hohen Sulfatanteilen, auftreten, da die Sulfatverbindung zu Schwefelwasserstoff reduziert wird und damit der gebildete H₂S-Gehalt den Prozeßablauf wesentlich beeinträchtigen kann:

Bei einer Abtötung oder Lähmung von Methanbakterien, also einer Störung der acetogenen-methanogenen Phase, wird weniger Wasserstoff abgebaut, d.h., der H₂ - Partialdruck steigt. Da dieser als Inhibitor für die acetogenen Bakterien wirkt, muß damit gerechnet werden, daß die Stoffwechselproduktion -insbesondere von Essigsäure- dieser Bakterien zum Erliegen kommt sowie durch die H₂-Anreicherung stärker reduzierte Produkte gebildet werden.

Bei einem biologischen Abbau von Schlempe muß mit einem Schwefelwasserstoffgehalt im Biogas von ca. 2,5 % gerechnet werden, das bedeutet, daß dieses Brenngas in einem Ottomotor ohne vorherige Elimination des H₂S-Anteiles nicht verbrannt werden kann.

Ein weiterer Nachteil besteht darin, daß bei relativ großen Verweilzeiten des Substrates nur wirtschaftliche Gasausbeuten von etwa 1 - 2 m³/d Biogas pro m³ Faulraum bzw. niedrige Abbaugrade der organischen Substanzen möglich sind.

Der Heizwert des Biogases beträgt bei einem Einstufenreaktor, der nach neuzeitlichen Verfahrenstechniken normale Substrate anaerob behandelt, 7 KWh/m³ bei einer Gaszusammensetzung von 70 % Methan (CH₄), 29,7 % Kohlendioxyd (CO₂) und 0,3 % Schwefelwasserstoff (H₂S).

Aufgrund der vorgenannten Erkenntnisse ist schon vorgeschlagen worden, eine biotechnologische Trennung in die Hydrolyse- und Säurephase sowie in die Methanbildungsphase vorzusehen, um für die entsprechenden Bakterienkulturen optimale Lebensbedingungen zu schaffen, vgl. DE-OS 31 02 739.

Es ist dabei von Bedeutung, daß die Säure- bzw. Methanbildungsphase nicht in zwei getrennt aufgestellten Reaktoren abläuft, da bei dieser Systemlösung die Bildung von Essigsäure durch den H₂ - Partialdruck beeinflußt wird, sondern daß die Hydrolyse- und Säurephase sich in einem kombinierten Reaktor abspielt. In diesem Zweistufen-Reaktor wird durch die anwesenden Methanbakterien der Wasserstoffpartialdruck im Säurereaktor entsprechend reduziert und damit der ph-Wert entsprechend eingestellt, wobei gleichzeitig mehr Essigsäure sowie eine optimale Zusammensetzung an besser abbaubaren Stoffwechselprodukten wie Essig-, Butter-,Capronsäure und Ethanol gebildet wird. Die Reduzierung des H₂ - Partialdruckes findet bei dieser Verfahrenstechnik inform einer Kopplungsreaktion -die auch als Stoffaustausch bezeichnet werden kann- durch Methanbakterien bzw. den universellen Wasserstoffträger NAD sowie anderer Coenzyme statt. Bei dieser Kopplungsreaktion ist der Diffusionsstrom von Wasserstoffmolekeln und Elektronen durch den Ringspalt maßgebend.

Ein weiterer konstruktiver Punkt ist die Aufteilung der Fermentationsräume I und II, also der Säure- bzw. Methanbildungsphase mit einem Volumenverhältnis von vorzugsweise 1: 10.

Es ist bekannt, daß in einer Versäuerungsstufe, je nach Milieubedingungen des Substrats, erheblich mehr Bakterien -bedingt durch die kurzen Generationszeiteder fakultativen Anaerobier- vorhanden sind, als in einem Methanreaktor. Durch diese Erkenntnis liegt ein unterschiedliches Produktionsverhältnis zwischen Säure- und Methanbakterien vor bzw. ist eine Raumbelastung des Hydrolyse- und Versäuerungs-Reaktors um mehr als das Zehnfache eines Einstufenreaktors möglich, ohne daß eine wesentliche Minderung der erzeugten Stoffwechselprodukte bzw. Abbaugrade in der Stufe I auftritt. Darüber hinaus kann bei einer hohen Raumbelastung mit ph-Wert-Einstellung eine weitgehende Stabilität der produzierten Stoffwechselprodukte erwartet werden. Auch die Zusammensetzung und Verteilung der produzierten Stoffwechselprodukte wie Essig-, Butter-, Valerian-, Capron-, Ameisen- und Milchsäure sowie Ethanol, Wasserstoff und Kohlendioxyd, kann in der Fermentationsstufe I bei der mesophilen Temperatur von 30°C im ph-Wertbereich von 5 bis 6 unter Anwendung des geschilderten Verfahrens sowie der fixierten Raumaufteilung und hohen Raumbelastung der Hydrolyse- und Versäuerungsstufe als optimal erwartet werden. Bei einem ph-Wert von 5 - 6 ist mit einer max. Wachstumsrate der säurebildenden Bakterien zu rechnen.

Die im Säurereaktor individuell produzierten Stoffwechselprodukte lassen sich im Methanreaktor schneller und besser in das Endprodukt Methangas und Kohlendioxyd umwandeln, wobei die Wasserstoffverwertenden Methanbakterien ihre volle Leistungsfähigkeit in der Fermentationsstufe II entfalten können. Bei diesem Verfahren sind die Stoffwechselleistungen der einzelnen Abbauphasen wesentlich höher als in einem Einstufenreaktor bzw. wird durch die geringere Kohlenstoff-Investition in die Bakterienkultur mehr Methan synthetisiert.

Darüber hinaus findet durch die verfahrenstechnische Prozeßführung im Methanreaktor eine vermehrte Adaption der symbiotischen Population von $CO_2$ verarbeitenden methanogenen Bakterienstämmen statt, d.h., daß die Bildung von Essigsäure in der Fermentationsstufe II gemäß nachstehender Gleichung erfolgt.

$$2 CO_2 + 4 H_2 \rightarrow CH_3COOH + 2 H_2O$$

Durch diese Konsequenz wird der $CO_2$-Partialdruck herabgesetzt und damit eine höhere Acetatumsetzung zu Methangas möglich.

$$CH_3COOH \rightarrow CH_4 + CO_2 - 32 E$$

Die Aufgabe der vorliegenden Erfindung besteht nun darin, ein Verfahren und eine Vorrichtung der eingangs genannten Art so zu verbessern, daß ein hoher Abbaugrad bei kurzen Verweilzeiten sowie ein größerer Anteil an Methan synthetisiert wird.

Diese Aufgabe wird verfahrensmäßig durch die Merkmale im Kennzeichen des Anspruchs 1 und vorrichtungsmäßig durch die Merkmale im Kennzeichen des Anspruchs 4 gelöst.

Durch die erfindungsgemäße Ausbildung findet eine kontinuierliche verbesserte Reduzierung des Wasserstoff-Partialdruckes über den Ringspalt bzw. der abgesetzten Produkte im ersten Reaktorraum statt, wobei durch die vorgesehene mechanische Umwälzung nach dem Kreisprinzip sich in der Hydrolyse- und Versäuerungsstufe ein gleichmäßiger Wasserstoff-Partialdruck aufbaut. Durch die Erfindung wird ein erhöhter Anteil an Essigsäure produziert und die weniger erwünschte Produktion von Propion- und Valeriansäure weitgehend verhindert. Bei dem erfindungsgemäß ausgebildeten Zweistufenverfahren können die Verweilzeiten der Substrate in dem kombinierten Reaktor mit Biogasleitungen $(CH_4 + CO_2)$ von 3-6$m^3$/d pro $m^3$ Faulraum verringert werden. Es wird ein hoher Abbaugrad der organischen Substanzen erreicht. Das produzierte Biogas ist fast völlig frei von dem unerwünschten Anteil an Schwefelwasserstoff.

Der erfindungsgemäße Zweistufenreaktor ist äußerst betriebssicher, wobei auch toxische Stoffe in Grenzen eintragbar und abbaubar sind.

Die mit der Erfindung erzielbaren Ergebnisse können noch weiter verbessert werden durch Überlagerung der hydraulischen Umwälzung mit einer weiteren Umwälzung durch thermische Zirkulation, wie dies in Anspruch 3 und 5 angegeben ist.

Vorteilhafte zweckmäßige Weiterbildungen der erfindungsgemäßen Vorrichung sind in den Unteransprüchen 5 bis 27 angegeben.

Bei der erfindungsgemäß vorgesehenen hydraulischen Umwälzung und thermischen Zirkulation im zweiten Reaktorraum findet eine Reduzierung des Wasserstoff-Partialdruckes, insbesondere im Sedimentationsbereich des ersten Reaktorraumes über den Ringspalt zwischen dem kegelförmigen Boden und dem Mischinjektor durch eine Wasserstoff-Zehrung von Methanbakterien sowie durch die Diffusion von Wasserstoffmolekeln in den Fermentationsraum 2 statt. Durch diese Verfahrenstechnik wird eine geringere Menge von Propion- und Valeriansäure gebildet und gleichzeitig ein erhöhter Anteil an Essigsäure sowie besser abbaubaren Stoffwechselprodukten mit einer optimalen Mengenverteilung produziert. Für die optimale Bildung der Produktpalette wie Essig-, Butter- und Capronsäure sowie Ethanol und Methanol bzw. Wasserstoff ist nach Beendigung der Beschickungsphase der am stillstehenden Substrat im Sedimentationsbereich (Grenzschichtfläche des Ringspaltes 11) des Reaktorraumes 1 vorbeiströmende Substratstrom aus dem Reaktorraum 2 maßgebend.

Bei der Beschickung des ersten Reaktorraumes strömen die produzierten Stoffwechselprodukte der ersten Fermentationsstufe durch den Ringspalt, werden mit Belebtschlamm der zweiten Fermentationsstufe des zweiten Reaktorraumes im Mischinjektor substratschonend, d.h. ohne Einwirkung von Scherkräften, durchmischt und über die von der zylindrischen Wand umschlossene zylindrische Kammer in den zweiten Reaktorraum im Kreise mit Rückvermischung gefördert, wodurch eine ausgezeichnete Verteilung der Stoffwechselprodukte für einen hochgradigen Abbau erreicht wird.

Die Strahlpumpe mit seitlich offener Düsenmischkammer saugt über den Wärmeaustauscher, der in seiner Doppelfunktion auch als Leitrohr dient, Substrat von unten aus dem ersten Fermentationsraum an, wobei das Verhältnis von Belebtschlamm zum eingespeisten frischen Substrat mindestens 3: 1 beträgt. Die Substratumwälzung erfolgt im Kreis mit Rückmischung. Gleichzeitig wird durch den Flüssigkeitsstrahl der Strahlpumpe eine eventuell gebildete Schwimmdecke aufgebrochen und aufgelöst. Darüber hinaus werden während der hydraulischen Umwälzung die im seitlichen Kegelboden sedimentierten Stoffwechselprodukte unwesentlich gestört bzw. aufgewirbelt. Der Wärmeaustauscher bewirkt eine zusätzliche Umwälzung des Substrates durch Konvektion.

Die doppelwandig ausgeführte Zylinderwand 8 hat zusätzlich die Funktion einer Wärmedämmung, insbesondere durch das im Zwischenraum eingeschlossene Biogas. Durch die Wärmedämmung wird erreicht, daß in den Reaktorräumen mit unterschiedlichen Substrattemperaturen gefahren werden kann.

Bei Biogas-Reaktoren mit einem Inhalt unter 300 m³ befindet sich der erste Reaktorraum in der Mitte des zweiten Reaktorraumes. Bei Biogas-Reaktoren mit einem Inhalt größer als 300 m³ werden normalerweise mehrere einzelne Reaktorräume für den ersten Fermentationsprozeß eingesetzt.

Durch den hochgezogenen Kegelboden des ersten Reaktorraumes können sich keine Sedimentationsschichten bilden. Die in den Reaktorraum 1 geförderten und nicht abbaubaren Stoffe rutschen auf dem Kegel in den zweiten Reaktorraum und können von dort abgeschlammt bzw. ausgetragen werden.

Durch das vorgesehene Doppel-U-Rohr zum Austragen der biologisch abgebauten Substanzen aus dem zweiten Reaktorraum ergibt sich die Möglichkeit einer Druckgas-Speicherung in Abhängigkeit von der U-Rohrschenkelhöhe.

Die Erfindung soll nachfolgend anhand der beigefügten Zeichnung näher erläutert werden.

Es zeigt:

Fig. 1 einen Schnitt durch einen erfindungsgemäß ausgebildeten Biogas-Reaktor,

Fig. 2 schematisch einen Horizontalschnitt durch einen erfindungsgemäßen Biogas-Reaktor mit mehreren Reaktorräumen für den ersten Fermentationsprozeß,

Fig. 3 schematisch die Ausbildung der bei den Biogas-Reaktoren nach Fig. 1 und 2 vorgesehenen Wärmetauschern und

Fig. 4 eine bevorzugte Ausbildung eines beim erfindungsgemäßen Biogasreaktors vorgesehenen Mischinjektors.

Die Zeichnung zeigt einen kombinierten Biogas-Reaktor mit einem ersten Reaktorraum 1 und einem zweiten Reaktorraum 2. Im ersten Reaktorraum, der innerhalb des zweiten Reaktorraumes angeordnet ist, findet ein erster Fermentationsprozeß (Hydrolyse- und Säurebildung) und im zweiten Reaktorraum ein zweiter Fermentationsprozeß (Methanbildung) statt.

Der erste Reaktorraum 1 weist einen hochgezogenen Boden mit kegelförmiger Bodenfläche 19 auf und wird gebildet von einem doppelwandigen Zylinder 8, zwischen dessen Wänden ein ringzylindrischer Zwischenraum 40 gebildet ist. Der Zylinder 8 endet oberhalb des kegelförmigen Bodens 19, so daß zwischen Zylinder 8 und Boden 19 ein Ringspalt 11 gebildet wird, über den der erste Reaktorraum 1 mit dem zweiten Reaktorraum 2 in offener Verbindung steht. Das untere Teil des doppelwandigen Zylinders 8 ist als Mischinjektor 10 ausgebildet.

Die Zylinderwand 8 wird von einem weiteren Zylinder 9 beabstandet umgeben, so daß eine zylindrische Ringkammer 44 gebildet wird, die als Überlaufkammer ausgebildet ist und beabstandet zum Boden des zweiten Reaktorraumes 2 endet, so daß ein weiterer Ringspalt 46 gebildet wird, der wie auch der Ringspalt 11 in eine Injektionsmischkammer 45 führt, die begrenzt wird vom Kegelboden 19, der zylindrischen Wand 9 sowie vom Mischinjektor 10. Über den Zwischenraum 40 kann Biogas unter Druck mittels eines Gasverdichters 5 dem Mischinjektor zugeführt werden. Der Saugstutzen des Gasverdichters 5 ist an einen Biogasraum 42 des Reaktorraumes 2 angeschlossen und der Druckstutzen des Gasverdichters ist mit dem Zwischenraum 40 des Doppelwandzylinders 8 verbunden.

Das untere Ende des Zylinders 9 ist als Wärmetauscher 12 ausgebildet. Mit Hilfe des Mischinjektors 10 wird eine hydraulische kreisumwälzung des Substrates im Reaktorraum 2 hervorgerufen, wie dies durch die Linie 48 angedeutet ist. Eine weitere thermische Zirkulation des Substrates wird durch den Wärmetauscher 12 hervorgerufen. Diese thermische Zirkulation überlagert die hydraulische Umwälzung.

Im Reaktorraum 1 ist oberhalb des Kegelbodens 19 zentral eine Strahlpumpe 7 mit seitlich offener Düsenmischkammer angeordnet, die das Substrat im Reaktorraum 1 von unten ansaugt und nach oben pumpt. Die Strahlpumpe 7 ist umgeben von der Wendel eines rohrförmigen Wärmetauschers 6, der damit als Leitrohr wirkt, wie auch die ringförmige Zwischenkammer 44, wodurch eine hydraulische Kreisumwälzung des Substrates im Reaktorraum 1 bewirkt wird, wie dies durch die Linien 50 angedeutet ist. Diese hydraulische Umwälzung wird überlagert von einer thermischen Zirkulation, die hervorgerufen wird durch den Wärmetauscher 6.

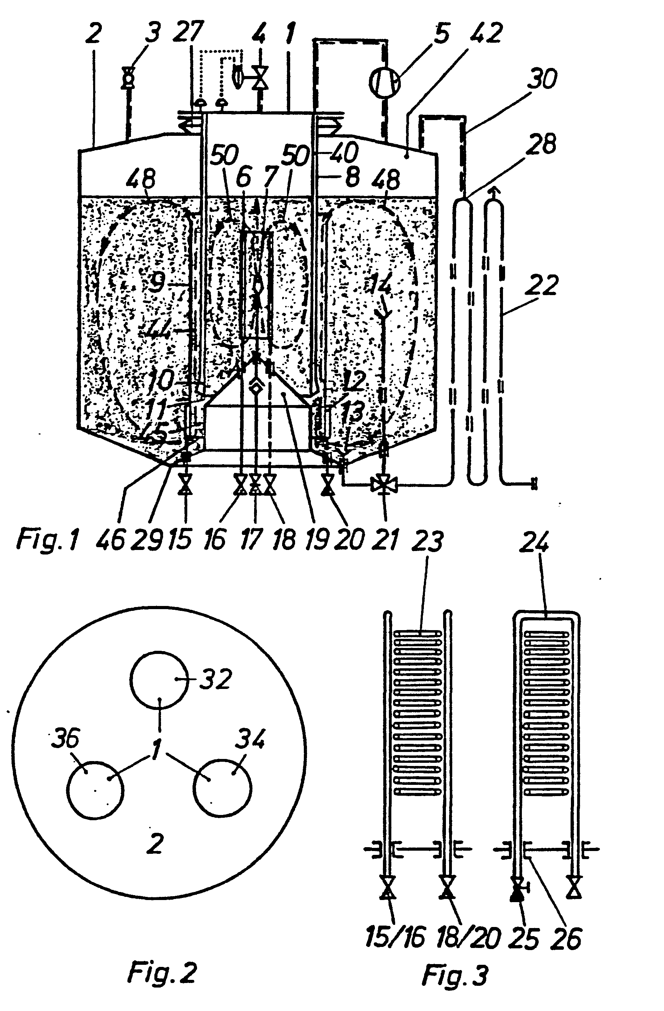

Die Vorlauf- und Rücklaufanschlüsse der Wärmetauscher 12 und 6 sind mit den Bezugszeichen 15 und 20 bzw. 16 und 18 bezeichnet. Die gewendelten Wärmetauscherrohre 23 bestehen vorzugsweise aus Kunststoff (beispielsweise Polypropylen) (Fig. 3). Als Stützkonstruktion dienen jeweils zwei einzelne U-förmige Rohre 24, die gleichzeitig Verteilerrohre für den Vorlauf und den Rücklauf bilden. Die Rohre werden mittels justierbarer Rohrbuchsen 26 durch die Reaktorwand geführt. Mit dem Bezugszeichen 25 ist ein Entlüftungsventil bezeichnet. Die im Fermentationsraum 2 biologisch abgebauten Substanzen sammeln sich in dem umlaufenden trichterförmigen Boden 29.

Im Reaktorraum 2 sind noch zwei Austragstutzen 13 und 14 angeordnet, die wahlweise über ein Drei-Wegeventil 21 mit einem Doppel-U-Rohr 22 verbindbar sind zum Austragen biologisch abgebauter Substrate. Austragstutzen 13 befindet sich in Bodennähe und der Austragstutzen 14 etwa in halber Höhe des Reaktorraumes 2. Der erste U-Rohr-Schenkel 28 ist über eine Leitung 30 mit dem Biogasraum 42 verbunden.

Das im Reaktorraum 2 entstehende Biogas $CH_4$ + $CO_2$ ist über ein Absperrorgan 3 und das im Reaktorraum 1 entstehende saure Biogas $CO_2$ + $H_2S$ ist über ein differenzdruckgeregeltes Überströmventil 4 ableitbar.

Bei Reaktoren mit einem Inhalt von weniger als 300 m³ wird ein einziger Reaktorraum für den ersten Fermentationsprozeß verwendet, der zentral im Reaktorraum 2 für den zweiten Fermentationsprozeß angeordnet ist, vgl. Fig. 1. Bei Reaktoren mit einem Inhalt von mehr als 300 m³ werden mehrere, beispielsweise drei einzelne Reaktorräume 32, 34, 36 für den ersten Fermentationsprozeß verwendet, die gleichmäßig verteilt im Reaktorraum 2 für den zweiten Fermentationsprozeß angeordnet sind, vgl. Fig. 2, wobei der Wirkungsbereich der einzelnen Reaktorräume so eingestellt wird, daß er bei 3 Reaktorräumen einen Aktionsradius aufweist, der etwa 25 % des Gesamtdurchmessers des Reaktors 2 ausmacht.

Der in der Zeichnung dargestellte Zweistufen-Reaktor ist in einem Raumverhältnis des ersten Reaktorraumes 1 für die Säurebildungsphase zum zweiten Reaktorraum 2 für die Methanbildungsphase von etwa 1 : 10 gebaut.

Der in der Zeichnung dargestellte Biogas-Reaktor arbeitet wie folgt. Der erste Reaktorraum 1 wird mit dem zu behandelnden Substrat über einen Absperrschieber 17 beschickt. Mit Hilfe der Strahlpumpe 7 wird das Substrat hydraulisch umgewälzt, wobei ein Verhältnis von Belebtschlamm zum eingespeisten frischen Substrat von mindestens 3 : 1 eingestellt wird. Die Beschickung erfolgt dabei über eine externe Pumpe, die nicht dargestellt ist. Diese Pumpe ist mit dem Absperrschieber 17 sowie der Strahlpumpe 7 verbunden. Während der Beschickung, die quasi-kontinuierlich erfolgt, wird mittels der Strahlpumpe 7, die an der Düsenmischkammer mit seitlichen Schlitzen versehen ist, Belebtschlamm angesaugt und mit dem eingespeisten Substrat gemischt. Dabei strömt während des Beschickungsvorganges Belebtschlamm von unten in den Wärmetauscher ständig nach. Nach der Beschickung des Reaktorraumes 1 wird durch den eingetretenen Temperaturabfall der Wärmetauscher 6 in Betrieb genommen, um die vorgesehene optimale Temperatur von ca. 30° C einzustellen und konstant zu halten. Durch die Einschaltuag des Wärmetauschers findet, wie bereits erwähnt, eine weitere Umwälzung inform einer thermischen Zirkulation des Substrates statt.

Während der Beschickung des Reaktorraumes 1 werden die im Kegelbodenbereich 19 angesammelten Stoffwechselprodukte über den Ringspalt 11 in die Injektionsmischkammer 45 gefördert und mit Belebtschlamm aus dem Reaktorraum 2 gemischt und mit Hilfe des Mischinjektors über die Zwischenringkammer 44 in den Reaktorraum 2 gefördert und durch gleichzeitige Kreisumwälzung entsprechend verteilt. Gleichzeitig erfolgt durch den Temperaturabfall im Reaktorraum 2, der mit einer optimalen Temperatur von ca. 35°C betrieben wird, die Einschaltung des Wärmetauschers 12. Auch im Reaktorraum 2 findet nach Abschaltung des Gasverdichters 5 bzw. dés Mischinjektors 10 eine thermische Umwälzung über den Zwischenraum 44 durch die Wirkung des Wärmetauschers 12 statt. Eine Anordnung aus Gasverdichter 5, ringförmigem Zwischenraum 40 als Leitrohr, Mischinjektor 10 und ringförmige Zwischenkammer 44 als weiteres Leitrohr wird auch als Mammutpumpe bezeichnet.

Durch die hydraulische und thermische Umwälzung des Substrates im Reaktorraum 2 wird über den Ringspalt 11 eine Reduktion des Wasserstoff-Partialdruckes im Sedimentationsbereich des Reaktorraumes 1 vorgenommen. Das im Reaktorraum 2 erzeugte Biogas ($CH_4$ + $CO_2$) wird über das Absperrorgan 3 entnommen und den Verbrauchern zugeführt. Das im Reaktorraum 1 erzeugte saure Biogas ($CO_2$ + $H_2S$) kann über das Regelventil 4 abgelassen werden.

Nach entsprechendem biologischen Abbau der organischen Substanzen sammeln sich diese im trichterförmigen Boden 29 bzw. wird der Faulschlamm aus dem Reaktorraum 2 über den Austragstutzen 13 oder 14 gemäß der in den Reaktorraum 1 eingespeisten frischen Substratmenge ausgetragen. Der ausgetragene Faulschlamm fließt durch das Doppel-U-Rohr 22, das gleichzeitig als Siphon für eine Druckgasspeicherung dient, durch den Anschluß an den Biogasraum 42 mit Hilfe der Verbindungsleitung 30.

Zur Erzielung eines günstigen Reaktorwirkungsgrades bei möglichst geringem Materialverbrauch wird ein Verhältnis zwischen Höhe und Durchmesser des Reaktors von etwa 2 : 1 gewählt.

Weiter kann der Ringspalt 11 mit Hilfe der Vorrichtung 27 nachträglich in der Höhe eingestellt werden.

Um bei verschiedenen Reaktorbelastungen -unter Berücksichtigung der im Fermentationsraum 2 mit Druck gespeicherten Gasmenge- ein konstantes Faulraumvolumen zu erzielen, wird durch eine Differenzdruckregelung, welche zwischen Fermentationsraum 1 und Fermentationsraum 2 erfolgt, das im Fermentationsraum 1 produzierte überschüssige Biogas ($CO_2 + H_2S$) durch das Überströmventil 4 abgelassen.

Für Sonderfälle kann die Ablassleitung des Überströmventiles 4 mit der Entnahmeleitung 3 zusammengeschaltet werden. In diesem Fall muß je nach Substratart das Gas über eine Gaswaschvorrichtung bzw. Entschwefelungsanlage gereinigt werden.

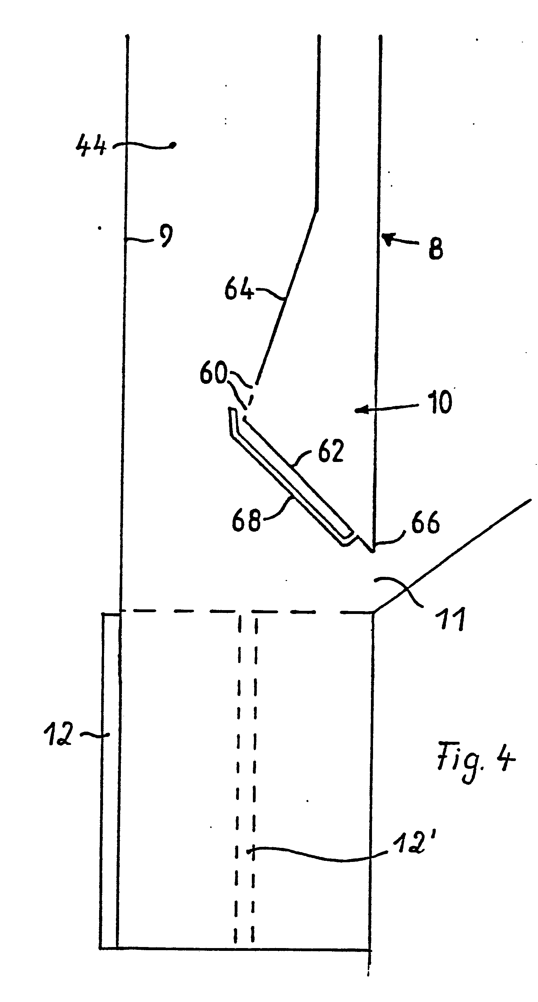

Wie schon oben erwähnt, ist der untere Teil des doppelwandigen Zylinders 8 als Mischinjektor 10 ausgebildet. Über diesen Mischinjektor wird Biogas unter Druck in den Reaktorraum 2 eingeblasen, und zwar über in den Reaktorraum führende Löcher 60. Der Mischinjektor wird gebildet durch den unteren Teil der inneren Wandung des Zylinders 8, einen unter einem Winkel zu dieser inneren Wandung nach oben in den Reaktorraum 2 zeigenden umlaufenden Boden 62 und durch ein von der äußeren Wand des Zylinders 8 unter einem Winkel nach außen in den Reaktorraum zeigendes umlaufendes Teil 64, das mit dem Boden 62 verbunden ist. Die Löcher 60 sind in diesem Teil 64 nahe der Verbindungsstelle mit dem Boden 62 ausgebildet. Der Winkel zwischen dem Boden 62 und der Innenwand des Zylinders 8 beträgt etwa 46° und der Winkel zwischen der Außenwandverlängerung des Zylinders 8 und dem Teil 64 etwa 18°. Durch diese Ausbildung wird ein im Querschnitt etwa dreieckförmiger ringförmiger Ringraum im unteren Teil des doppelwandigen Zylinders 8 als Mischinjektor gebildet. Die Löcher 60 sind umfangsmäßig verteilt angeordnet. Es können mehrere Lochreihen mit zueinander versetzt angeordneten Löchern vorgesehen werden. In der Zeichnung (Fig. 4) sind zwei Lochreihen schematisch angedeutet. Die Löcher weisen vorzugsweise einen Durchmesser von etwa 4 mm auf. Vom unteren spitzwinklig zulaufenden Ende 66 des Mischinjektors sind mehrere Entwässerungsrohre 68 nach oben geführt, die im Boden 62 münden und etwa in Höhe der Löcher 60 enden, vorzugsweise mit geringem Abstand zu den Löchern.

Der Wärmeaustauscher 12 kann insbesondere bei größeren Anlagen, bei denen die zylindrische Ringkammer 44 eine ausreichende lichte Weite hat, sich in axialer Richtung erstreckend zentral im unteren Teil der zylindrischen Ringkammer 44 angeordnet sein, wie dies gestrichelt in der Fig. 4 eingezeichnet ist, vgl. Wärmetauscher 12'. Durch diese Anordnung kann die thermische Zirkulation verbessert werden. Außerdem verringert sich hierdurch die statische Belastung des Wärmetauschers durch den Zylinder 9.

**Patentansprüche**

1. Verfahren zur anaeroben Behandlung von Substraten mit organischen Stoffen, insbesondere mit einem hohen Anteil an hochmolekularen organischen Stoffen wie Proteinen, Kohlehydraten und Fetten, zur Erzeugung von Biogas, bei dem in einem im ersten Reaktorraum stattfindenden Fermentationsprozeß die Substrate durch fermentative und säurebildende Bakterien vorzugsweise in Essigsäure, Wasserstoff und Kohlendioxid überführt werden und dann die entstehenden Produkte über eine offene Verbindung in einen zweiten Reaktorraum gelangen und dort durch acetogene und methanogene Bakrerien in einem zweiten Fermentationsprozeß in Biogas (Methan und Kohlendioxid) umgewandelt werden, dadurch gekennzeichnet, daß das zu behandelnde, jeweils eingespeiste, frische Substrat zusammen mit Belebtschlamm im ersten Reaktorraum nach dem Kreisprinzip mit Rückvermischung umgewälzt wird und daß die im ersten Fermentationsprozeß entstandenen Produkte mit Belebtschlamm des zweiten Fermentationsprozesses vermischt und ebenfalls nach dem Kreizprinzip mit Rückvermischung umgewälzt werden, wobei die Durchmischung ohne Einwirkung von Scherkräften, d.h. ohne Rührwerk erfolgt.

2. Verfahren nach Anspruch 1, dadurch gekennzeichnet, daß das Verhältnis von frischem Substrat Belebtschlamm wenigstens 1 : 3 beträgt.

3. Verfahren nach Anspruch 1, dadurch gekennzeichnet, daß der Umwälzung eine weitere Umwälzung durch thermische Zirkulation überlagert wird/oder folgt.

4. Vorrichtung zur Durchführung des Verfahtens nach einem der vorhergehenden Ansprüche mit einem Bioreaktor, der einen ersten Reaktorraum aufweist für die Überführung der Stoffwechselprodukte aus einem organschen Substrat in erster Linie in Essig-, Butter- und Capronsäure sowie Wasserstoff und Kohlendioxyd und einen zweiten Reaktorraum zur Biogas (Methan und Kohlendioxyd), dadurch gekennzeichnet, daß im ersten Reaktorraum (1) und/oder im zweiten Reaktorraum (2) eine Einrichtung zur mechanischen bzw. hydraulischen Umwälzung vorgesehen ist.

5. Vorrichtung nach Anspruch 4, dadurch gekennzeichnet, daß weiterhin im ersten Reaktorraum (1) und/oder im zweten Reaktorraum (2) eine Einrichtung zur thermischen Umwälzung der in den Reaktorräumen befindlichen Substanzen vorgesehen ist zur Erzielung höherer Umwälzleistungen.

6. Vorrichtung nach Anspruch 4, dadurch gekennzeichnet , daß der erste Reaktorraum (1) mit einer doppelwandigen Zylinderwand (8) ausgebildet ist, über deren Zwischenraum (40) Biogas unter Druck von oben zuführbar ist und deren unteres Ende als Mischinjektor (10) ausgebildet ist für die Durchmischung der im Reaktorraum (1) erzeugten Produkte mit Belebtschlamm des zweiten Reaktorraumes (2) und zur Förderung der Mischung in den zweiten Reaktorraum.

7. Vorrichtung nach Anspruch 6, dadurch gekennzeichnet, daß die Förderung der Mischung über eine als Überlaufkammer ausgebildete ringförmige zylindrische Kammer erfolgt, die von der Zylinderwand (8) und einer diese beabstandet umgebenden zylindrischen Wand (9) gebildet wird, die mit Abstand oberhalb des Bodens des Reaktorraumes (2) endet.

8. Vorrichtung nach Anspruch 6 oder 7, dadurch gekennzeichnet, daß der erste Reaktorraum (1) einen hochgezogenen Boden mit kegelförmiger Bodenfläche (19) aufweist, daß zwischen dem unteren Ende der Zylinderwand (8) und dem kegelförmigen Boden (19) ein Ringspalt (11) gebildet ist, über den die im Reaktorraum (1) erzeugten Produkte in eine Injektionsmischkammer (45) gelangen, die begrenzt wird vom kegelförmigen Boden (19), dem unteren Teil der zylindrischen Wand (9) und dem Mischinjektor (10).

9. Vorrichtung nach einem der Ansprüche 6 bis 8, dadurch gekennzeichnet, daß im ersten Reaktorraum zur kreisförmigen Substratumwälzung eine Strahlpumpe (7) angeordnet ist, die eine seitlich offene Düsenmischkammer aufweist.

10. Vorrichtung nach Anspruch 9, dadurch gekennzeichnet, daß die Strahlpumpe (7) innerhalb eines senkrecht angeordneten rohrförmigen Wärmetauschers (6) angeordnet ist.

11. Vorrichtung nach Anspruch 10, dadurch gekennzeichnet, daß der Wärmetauscher U-förmige Vorlauf- und Rücklaufrohre (24) aufweist, die als Stützkonstruktion, Verteilerrohre und Entlüftungsrohre dienen und zwischen denen eine Rohrwendel (23) angeordnet ist.

12. Vorrichtung nach Anspruch 8, dadurch gekennzeichnet, daß der untere Teil der zylindrischen Wand (9) durch einen Wärmetauscher (12) gebildet wird oder daß zentral im unteren Teil der zylindrischen, von den Zylindern (8) und (9) gebildeten Ringkammer (44) ein Wärmetauscher (12') angeordnet ist.

13. Vorrichtung nach einem der Ansprüche 4 bis 12, dadurch gekennzeichnet, daß der zweite Reaktorraum (2) den ersten Reaktorraum (1) ringförmig umgibt.

14. Vorrichtung nach einem der Ansprüche 4 bis 13, dadurch gekennzeichnet, daß der erste Reaktorraum (1) aus mehreren einzelnen zylindrischen Reaktorteilräumen besteht, die innerhalb des zweiten Reaktorraumes (2) angeordnet sind.

15. Vorrichtung nach Anspruch 14, dadurch gekennzeichnet, daß drei gleiche, einzelne zylindrische Reaktorteilräume (32, 34, 36) vorgesehen sind.

16. Vorrichtung nach Anspruch 15, dadurch gekennzeichnet, daß die einzelnen zylindrischen Reaktorteilräume so aufgeteilt sind, daß der Wirkungsbereich jedes Reaktorteilraumes einen Radius aufweist, der etwa 25 % des Durchmessers des zweiten Reaktorraumes (2) ausmacht.

17. Vorrichtung nach einem der vorhergehenden Ansprüche, dadurch gekennzeichnet, daß der Reaktorraum (1) mit seiner Zylinderwand (8) und gegebenenfalls der zylindrischen Wand (9) als Einsatz ausgebildet und zusammen mit den Wärmeaustauschern (6 und 12) nach oben herausziehbar ist.

18. Vorrichtung nach einem der Ansprüche 4 bis 17, dadurch gekennzeichnet, daß das biologisch abgebaute Substrat über einen Austragstutzen (13) in Bodennähe oder einen Austragstutzen (14) im mittleren Bereich des zweiten Reaktorraumes (2) ausgetragen wird.

19. Vorrichtung nach einem der Ansprüche 4 bis 18, dadurch gekennzeichnet, daß das biologisch abgebaute Substrat über ein Doppel-U-Rohr (22) ausgetragen wird, dessen erster Schenkel (28) zur Erzielung einer Druckgasspeicherung im Biogasraum in Abhängigkeit von der Höhe des Schenkels über eine Leitung (30) mit dem Biogasraum (42) des zweiten Reaktorraumes (2) verbunden ist.

20. Vorrichtung nach Anspruch 6, dadurch gekennzeichnet, daß das Biogas mit Hilfe eines Gasverdichters (5) zugeführt wird, dessen Druckstutzen an den Zwischenraum (40) und dessen Saugstutzen an den Biogasraum (42) des Reaktorraumes (2) angeschlossen ist.

21. Vorrichtung nach einem der Ansprüche 4 bis 20, dadurch gekennzeichnet, daß das Verhältnis des Volumens des ersten Reaktorraumes (1) zum Volumen des zweiten Reaktorraumes (2) 1 : 2 bis 1 : 20, jedoch vorzugsweise 1 : 10 beträgt.

22. Vorrichtung nach einem der Ansprüche 4 bis 21, dadurch gekennzeichnet, daß die Temperatur im Reaktorraum (2) auf etwa 35° Celsius und im Reaktorraum (1) auf etwa 30° Celsius eingestellt wird.

23. Vorrichtung nach Anspruch 22, dadurch gekennzeichnet, daß die Temperaturen mit Hilfe der Wärmeaustauscher (6 und 12) eingestellt werden, wobei die Temperatur mit Hilfe von Sensoren überwacht wird.

24. Vorrichtung nach einem der vorhergehenden Ansprüche, gekennzeichnet durch Anwendung bei Batch-Prozessen in Brauereien, Brennereien und in der pharmazeutischen Industrie.

25. Vorrichtung nach einem der vorhergehenden Ansprüche, dadurch gekennzeichnet, daß das Verhältnis Höhe:Durchmesser des Biogasreaktors etwa 2 : 1 beträgt.

26. Vorrichtung nach Anspruch 8, dadurch gekennzeichnet, daß der Ringspalt (11) durch eine Vorrichtung (27), die mit dem Reaktorraum (1 und 2) verbunden ist, in der Höhe eingestellt

27. Vorrichtung nach einem der Ansprüche 8 bis 14, dadurch gekennzeichnet, daß der Boden im Reaktorraum (2) inform einer umlaufenden Trichterpitze (29) ausgebildet ist.

28. Vorrichtung nach einem der Ansprüche 4 bis 26, dadurch gekennzeichnet, daß durch ein Überströmventil (4) gleiche Gasdrücke im Reaktorraum 1 und Reaktorraum 2 eingeregelt werden.

29. Vorrichtung nach Anspruch 6 oder 8, dadurch gekennzeichnet, daß der Mischinjektor (10) durch das untere Teil der zylindrischen Innenwand des doppelwandigen Zylinders (8), durch einen unter einem spitzen Winkel vom Ende der zylindrischen Innenwand nach oben in den Reaktorraum 2 zeigenden umlaufenden Boden (62) und ein unter einem spitzen Winkel von der zylindrischen Außenwand des Zylinders (8) nach unten in den Reaktorraum 2 zeigendes umlaufendes Teil (64) gebildet wird, das mit dem Boden (62) verbunden ist und in dem umlaufend mehrere Löcher (60) ausgebildet sind.

30. Vorrichtung nach Anspruch 29, dadurch gekennzeichnet, daß die Löcher (60) kurz oberhalb der Verbindungsstelle des Teiles (64) mit dem Boden (62) angeordnet sind.

31. Vorrichtung nach Anspruch 29 oder 30, dadurch gekennzeichnet, daß mehrere beabstandete Lochreihen mit versetzt zueinander angeordneten Löchern vorgesehen sind.

32. Vorrichtung nach Anspruch 29, dadurch gekennzeichnet, daß im spitzwinklig zulaufenden Ende (66) des Mischinjektors (10) mehrere Entwässerungsrohre (68) angeordnet sind, die im Boden (62) münden, außerhalb des Bodens (62) nach oben geführt sind und in der Höhe der Löcher (60) enden.

33. Vorrichtung nach Anspruch 29, dadurch gekennzeichnet, daß der Winkel zwischen dem Boden (62) und der zylindrischen Innenwand des Zylinders (8) etwa 46° beträgt und der Winkel zwischen der Verlängerung der zylindrischen Außenwand des Zylinders (8) und dem Teil (64) etwa 18°.

## Claims

1. A method for an anaerobic treatment of bases with organic elements, especially with a high portion of high-molecular organic elements, such as proteins, carbohydrates and fats to produce sewer gas. The fermentation process of the base takes place in the first chamber by means of fermentative- and acid-forming bacteria which will be transformed preferentially into sewer acetic acid, hydrogen and $CO_2$. After this, the products come through an open connection into the second reaction chamber where they are transformed into sewer gas by means of acetonic and methanic bacteria in a second fermentation process. The method is mainly characterized by the fact that the fresh base is circulated together with organic mud in the first reaction chamber according to the rotation principle with remixation. The products which are produced in the first fermentation process are mixed with the organic mud of the second fermentation process and then they are also circulated according to the rotation principle with remixation, whereby the mixing-up is effected without any influence of shear powers (stirrer).

2. A method according to claim 1.
The proportion of the fresh base to the organic mud is at least 1 : 3.

3. A method according to claim 1.
The circulation is superimposed by an other circulation by means of thermical circulation.

4. Device to realize the method according to the given claims concerning the sewer gas reactor which has a first reactor chamber for transplantation of metabolism products out of an organic base into acetic acid, butyric acid and capron acid as well as hydrogen and $CO_2$ and in a second reactor chamber to the transplantation of the products which had been produced in the first process - providing that the first reactor chamber and/or the second reactor chamber has a setting of a mechanical or hydraulical circulation.

5. Device according to claim 4. The first reactor chamber has to be equiped with a double-wall cylinder (8) over whose intermediate space sewer gas can be led in under pressure to the top and whose lower end has the function of a mix-injector (10). The mix-injector is used as mixer for the products - which are produced in reactor chamber (1) - and the organic mud of the second reactor chamber has also the function to increase the mixing-up in the second reactor chamber.

6. Device according to claim 4, characterized by the fact that the first reactor chamber and/or the second reactor chamber has a setting for a thermical circulation for the substances which are in the reactor chambers in order to reach maximum circulation effiency.

7. Device according to claim 6. The mixing-up is effected through a ringlike cylindrical chamber (spillway) which is formed between two walls of cylinder (8) and (9) and which ends above the bottom of reactor chamber 2.

8. Device according to claim 6 or 7. The reactor chamber (1) has a high bottom with a conical bottom expanse (19) whereby between the lower end of the wall of cylinder (8) and the conical bottom (19) a split-ring (11) is formed-over which the products - which are produced in reactor chamber (1) - are led into a injection mix chamber 45, which is surrounded by a conical bottom 19, the lower part of the cylindrical wall and the mix injector (10).

9. Device according to a claim from 6 to 8. In the first reactor chamber a stream pump has a laterally opened injection mix chamber. The stream pump is installed for the circular base rotation.

10. Device according to claim 9. The stream pump (7) is installed in the vertically placed tubular heat exchanger (6).

11. Device according to claim 10. The heat exchanger has forward motion pipes and return pipes which are U-formed and which have as support several distribution pipes and breather tubes. The connection is formed through a tubular coil (23).

12. Device according to claim 8. The lower part of the cylindrical wall (9) is formed by a heat exchanger (12) or the heat exchanger is situated centrally in the lower part of the cylindrical ring chamber between cylinder (8) and (9).

13. Device according to a claim 4 to 12. The second reactor chamber is surrounded by the first reactor chamber.

14. Device according to a claim from 4 to 13. The first reactor chamber consists of several individual cylindrical reactor-part-chambers which are to be found within the second reactor chamber.

15. Device according to claim 14. Three equal and individual cylindrical reactor-part-chambers are provided for.

16. Device according to claim 15. The individual cylindrical reactor part chambers are divided up in such a way, that the effiency of each reactor-part-chamber has a radius which means about 25 % of the diameter of the second reactor chamber.

17. Device according to the previous claims. The reactor chamber (1) with its cylindrical wall (8) and eventually the cylindrical wall (9) are replacable and can be drawn out to the top together with the heat exchanger.

18. Device according to a claim from 4 to 17. The biologically decomposable bases are carried away over an extracting connection pipe (13) near the bottom or over an extracting connection pipe in the middle of the second reactor chamber.

19. Device according to a claim from 4 to 18. The biologically decomposable bases are carried away over a double-U-pipe (22) whereby the first thigh (28) is connected to the sewer gas chamber (42) of the second reactor chamber (2) to reach a pressure gas storage in the sewer gas chamber in connection with the height of the thigh over the pipe (30).

20. Device according to claim 6. The sewer gas is led in with the help of a gas compressor 5, whereby a pressure fitting is connected to the intermediate chamber (40) and the suction fitting is connected to the sewer gas chamber (42) of the reactor chamber (2).

21. Device according to a claim from 4 to 20. The proportion of the volume of the first reactor chamber to the second reactor chamber is 1 : 2 up to 1 : 20, but preferentially 1 : 10.

22. Device according to a claim from 4 to 21. The temperature in the reactor chamber (2) is about 35° C and in reactor chamber (1) about 30° C.

23. Device according to claim 22. The temperatures are adjusted with the help of the heat exchangers (6) and (12), whereby the temperature is controlled with the help of sensors.

24. Device according to one of the previous claims. Use of the batch-process in breweries, distilleries and in the pharmaceutical industry.

25. Device according to one of the previous claims. The proportion of height and diameter of the sewer gas reactor is about 2 : 1.

26. Device according to claim 8. The split-ring 11 can be adjusted referring to the height by means of a device which is connected to reactor chamber (1) and (2).

27. Device according to claims 6 to 14, characterized in that the bottom of the reactor chamber (2) is shaped in the form of a surrounding funnel tip (29).

28. Device according to one of the claims 4 to 26, characterized in that by means of a drain valve (4) the same gas pressures can be adjusted in reactor chamber (1) and reactor chamber (2).

29. Device according to claim 6 or 8, characterized in that the mixinjector (10) is formed by the lower part of the cylindrical inner wall of the doble-walled cylinders (8), b y a circular bottom (62) extending in an acute angle from the end of the cylindrical inner wall upwardly into the reactor chamber (2) and by a surrounding part (64) extending in an acute angle from the cylindrical outer wall of the cylinder (8) downwardly into the reactor chamber (2) and connected to the bottom (62) and having several holes (60) in a circular arrangement.

30. Device according to claim 29, characterized in that the holes (60) are provided immediately above the point of connection of the part (64) to the bottom (62).

31. Device according to claim 29 or 30, characterized in that several rows of holes arranged in a distance to each other and staggered to each other are provided.

32. Device according to claim 29, characterized in that several dehydration pipes (68) are provided in the acute end (66) of the mixinjectors (10), which dehydration pipe (68) opening in the bottom (62) and running upwardly outside of the bottom (62) and ending in the height of the holes (60).

33. Device according to claim 29. The angle between the bottom (62) and the cylindrical internal wall of the cylinder (8) is about 46° and the angle between the extension of the cylindrical external wall of the cylinder and item (64) is about 18°.

## Revendications

1. Une méthode pour le traitement anaérobie des bases avec des éléments organiques, particulièrement avec une grande portion d'elements organiques qui sont ultra-moléculairs comme des protéins comme des protéins, des hydrates de carbone et des matières grasses pour produire de gaz de pourriture. Dans une première chambre de réaction la première phase de fermentation prend place et des bases sont transformées par moyen des microbes à l'acide acétique, l'hydrogène et l'oxygène l'oxyde de carbone. Les produits sont conduits par une connexion ouverte dans la deuxième chambre de réaction ou ils sont transformés à l'aide des microbes méthanique et acétiques dans la deuxième phase de fermentation au gaz de pourriture (la méthane et l'oxyde de carbone). La base est récuperée dans la première chambre de réaction avec la bourbe organique selon le principe de la circulation et des produits de la première phase de la fermentation sont mélangés avec la bourbe organique de la première phase da la fermentation. La mixtion est effectuée sans des forces de cisaillement (l'agitateur).

2. Une méthode selon titre 1. La proportion de la base à la bourbe organique est au moins 1 : 3.

3. Une méthode selon le titre 1. Le bouleversement est superposé d'un autre bouleversement par moyen d'une circulation thérmique.

4. Dispositif pour l'exécution de ce procédé qui a une chambre de réaction 1 pour la transformation des produits de métabolisme a l'acide acétique, l'acide butyrique et l'acide de capron ainsi que l'hydrogène et l'oxyde de carbone et une deuxième chambre de réaction pour la transformation des produits de la première chambre de réaction pour obtenir le gaz de pourriture. Dans la première et/ou dans la deuxième chambre de réaction, une circulation hydraulique prend place.

5. Dispositif selon titre 4. Dans la première chambre et/ou dans la deuxième chambre, une circulation thérmique prend place, pour obtenir une circulation améliorée.

6. Dispositif selon titre 4. Dans la première chambre il y a un cylindre avec une double-paroi 8. Le gaz de pourriture est conduit par la chambre intermédiaire 40. L'injecteur de mélange 10 mélange les produits de la première chambre de réaction avec la bourbe organique de la deuxième chambre.

7. Dispositif selon titre 6. Le transport des produits est effectué par une chambre de trop-plein qui est cylindrique et qui est formée par la paroi cylindrique 9 (qui finit près du fond de la première chambre).

8. Dispositif selon titre 6 ou 7. La première chambre a un fond éléve avec un fond conical 19. Entre la fin inférieure de la paroi du cylindre 8 et le fond conical 19, il y a une fente annulaire par lequel les produits de la première chambre peuvent être conduits dans la chambre d'injection 45, qui est limitée par le fond conical 19, la partie inférieure de la paroi cylindrique 9 et l'injecteur de mélange.

9. Dispositif selon l'un des titres 6 à 8. Dans la première chambre, il y a une pompe à jet (7) - pour la circulation de la base - qui a une chambre de mélange de tuyère qui est latérallement ouverte.

10. Dispositif selon titre 9. La pompe à jet (7) est située a l'intérieur de l'échangeur de chaleur annulaire (6).

11. Dispositif selon titre 10. L'échangeur de chaleur à des U-tubes de marche avant et de retour (24) qui ount la fonction d'un support de la distribution et de l'amorcage.

12. Dispositif selon titre 8. La partie inférieure de la paroi du cylindre (9) est formée par un échangeur de chaleur (12) ou l'échangeuer de chaleur (12) qui se trouve entre le cylindre (8) et (9) (dans une chambre annulaire qui est formée).

13. Dispositif selon l'un des titres 4 à 12. La première chambre consiste de plusieurs chambres de réaction partielles cylindriques qui sont situées à l'intérieure de la deuxième chambre.

14. Dispositif selon l'un des titres de 4 à 13. La deuxième chambre entoure la première chambre.

15. Dispositif selon titre 14. On forme 3 chambres de réaction partielles qui sont cylindriques et uniques (32, 34, 36)

16. Dispositif selon titre 15. Les trois chambres de réaction partielles sont partagées de cette manière que le rayon d'action de chaque chambre signifie 25 % du diamètre de la deuxième diamètre de réaction.

17. Dispositif selon l'un des titres précédents. La chambre de réaction avec la paroi de cylindre (8) et - éventuellement - la paroi cylindrique (9) peuvent être échangées avec l'échangeur de chaleur (6) et (12).

18. Dispositif selon l'un des titres de 4 à 17. La base décomposable est déchargée par une tubulure de déchargement (13) qui est située près du found ou par une autre tubulure de déchargement qui est au milieu de la deuxième chambre de réaction.

19. Dispositif selon l'un des titres 4 à 18. La base décomposable est déchargée par le double-U-tube (22) qui est connecté avec la chambre de gaz de purriture (42) de la deuxième chambre de réaction pour obtenir une accumulation du gaz comprimé dans la chambre de gaz de pourriture en dépendance de l'hauteur de la branche.

20. Dispositif selon titre 6. Le gaz de pourriture est conduit par moyen d'un compresseur de gaz. La tubulure de pression du compresseur est connectée avec la chambre intermédiaire (40) et la tubulure d'aspiration est connectée avec la chambre de gaz de pourriture (42) de la deuxième chambre de réaction.

21. Dispositif selon l'un des titres 4 à 20. La proportion du volume de la première chambre au volume de la deuxième chambre est 1 : 2 à 1 : 20, de préférence 1 : 10.

22. Dispositif selon l'un des titres 4 à 21. La température dans la deuxième chambre est ajustée a 35°C et dans la première chambre a 30°C.

23. Dispositif selon titre 22. Les températures sont ajustées à l'aide de l'échangeur de chaleur (6) et (12) et la température est surveillée par moyen d'un sensor.

24. Dispositif selon l'un des titres précédents. L'utilisation des procédés de batch dans les distilleries, les brasseries et dans l'industrie pharmaceutique.

25. Le mécanisme selon l'un des titres précédents. La proportion de l'hauteur: diamètre du réacteur de gaz de pourriture est 2 : 1.

26. Dispositif selon titre 8. Le fente annulaire (11) peut être ajusté par moyen d'un mécanisme 27 qui est connecté avec la chambre de réaction (1) et (2).

27. Dispositif selon l'un des titres 6 à 14, caracterisé par le fait que le sol dans le reactent (2) est construit dans la forme d'une pointe d'entonnier tournante (29).

28. Dispositif selon l'un des titres 4 à 26, caracterisé par le fait que a l'aude d'une valve de débordement (4) il est possible de régler la même pression de gaz dans le réactent (1) et le reacteur (2).

29. Dispositif selon l'un des titres 6 ou 8, caracterisé pour le fait que l'enjecteur de mélange (10) est formé par la partie basse de la paroi interne du cylindre à double parois (8), par le sol (62) qui est formé comme un angle pointu qui monte vers le reacteur (2) et qui est relié au sol et dans lequel qui est formé par le fond tournant (62) qui conduit à la deuxième chambre de reaction et par la partie tournante (64) - qui est connectée avec le fond (62) et dont lequel plusieurs trous (60) sont formés d'une facon tournante et qui conduit d'une facon acutangle de la paroi exterieur de cylindre (8) en bas dont la deuxième chambre de réaction.

30. Dispositif selon titre 29, caracterisé pour le fait les trous (60) sont disposés un peu au dessus de la partie de liaison de la piece (64) avec le sol (62).

31. Dispositif selon l'un des titres 29 ou 30, caracterisé pour le fait que plusieurs rangées de trous sont alignées en décalage au rangées de trous.

32. Dispositif selon titre 29, caracterisé par le fait que dans la terminaison à angle pointu (66) de l'enjecteur à mélange (10) sont alignés plusieuers tuyau de drainage (68) qui se déversent dans le sol (62), qui se dirigent à l'extérieur du sol (62) vers le haut; et qui se verminent à la hauteur des trous (60).

33. Dispositif selon titre 29. L'angle entre le fond (62) et la paroi intérieure cylindrique du cylindre (8) est 46° et l'angle entre l'allongement de la paroi extérieure cylindrique du cylindre (8) et la partie (64) est 18°.

0 172 443

Fig.1  46 29 15  16  17 18  19 20  21 23  24

Fig.2     Fig.3

15/16 18/20 25 26

*Fig. 4*